# FASCICULE DE BREVET EUROPEEN

(11) **EP 0 565 449 B1**
(45) Date de publication et mention de la délivrance du brevet: **02.01.1997**
(21) Numéro de dépôt: 93400918.4
(22) Date de dépôt: 08.04.1993
(51) Int. Cl.: C07J 51/00, A61K 31/565, G01N 33/74, A61K 51/04

(54) **Marquage des hormones par le Rhenium et le technetium**
Rhenium- und Technetium Markierung von Hormonen
Rhenium and Technetium labelling of hormones

(30) Priorité: 10.04.1992 FR 9204411
(43) Date de publication de la demande: 13.10.1993
(73) Titulaire: LA REGION WALLONNE, B-5100 Namur (BE)
(72) Inventeur: Top, Siden, F-91090 Lisses (FR); Vessieres, Anne, F-94240 L'Hay-les-Roses (FR); Jaouen, Gérard, f-94240 L'Hay-les-Roses (FR); Quivy, Jacques, B-1348 Louvain-la-Neuve (BE)
(74) Mandataire: Warcoin, Jacques

(56) Documents cités:
- EP-A- 0 105 785
- EP-A- 0 345 153
- EP-A- 0 441 491
- WO-A-92/14493
- JOURNAL OF ORGANOMETALLIC CHEMISTRY vol. 414, no. 1, 13 Août 1991, LAUSANNE CH pages C22 - C27 S. TOP ET AL 'Reactions of eq,eq-Re2(CO)8(MeCN)2 with Phenylacetylene and alpha-Ethynylestradiol. A New Synthesis of Acetylide Complexes'
- PURE AND APPLIED CHEMISTRY vol. 57, no. 12, Décembre 1985, LONDON GB pages 1865 - 1874 G. JAOUEN ET AL 'Transition Metal Carbonyl Oestrogen Receptor Assay'
- ORGANOMETALLICS vol. 6, no. 9, Septembre 1987, US pages 1985 - 1987 G. JAOUEN ET AL 'Transition-Metal Carbonyl Clusters as Novel Infrared Markers for Estradiol Receptor Site Detection'
- ORGANOMETALLICS vol. 11, no. 3, 1992, US pages 1201 - 1209 S. TOP ET AL 'Synthetic, Structural, and Reactivity Studies of Di-Rhenium Carbonyl Complexes of 17-alpha-Ethynylestradiol and Phenylacetylene: Variable -Temperature Carbon-13- NMR Spectra and X-Ray Crystal Structure of (mu-H)(mu-C:C-R)Re2(CO)7(MeCN)'
- JACS vol 117, pp 8372-8380 (1995);
- J. Nuclear Medicine Vol 36 p 8S-13S (1995);
- J.Med.Chem.vol27(10)pp1287-91(1984)
- Römps Lexikon der Chemie

## Description

La présente invention concerne de nouveaux complexes organo-métalliques d'hormones stéroïdes, en particulier des complexes d'oestrogènes. La présente invention concerne également l'utilisation de la propriété de ligand spécifique de récepteur d'hormones stéroïdes, notamment d'oestrogène, des complexes selon l'invention, pour une application à titre d'agents d'imagerie lorsqu'ils comportent un isotope radioactif approprié pour métal ou une utilisation comme médicament, en particulier pour le traitement de cancers hormonaux dépendants. Dans ce dernier cas, lorsque le métal est un isotope radioactif approprié, les complexes peuvent être utilisés dans le cadre du concept de radiothérapie ciblé in-situ.

Les hormones stéroïdes se lient avec une haute affinité aux récepteurs protéiques situés, dans le cas des oestrogènes, dans le noyau des cellules cibles. Après liaison de l'hormone, il se produit un phénomène d'activation qui les rend capables de se lier au DNA et déclenche l'activation de la transcription des portions du génome qui contrôlent l'activité physiologique propre à l'hormone. Etant donné le passage du complexe stéroïde-récepteur à proximité du DNA, un isotope radioactif porté par le stéroïde pourra endommager gravement le DNA et avoir un effet léthal sur la cellule cible.

Or un certain nombre de cancers présentent une concentration élevée en récepteurs spécifiques aux oestrogènes. Il s'agit notamment des cancers du sein, de l'utérus, de l'ovaire et de la prostate. Par exemple, 65% des cancers du sein présentent des niveaux détectables de récepteurs d'estrogènes (de 5000 à 50000 molécules de récepteurs par cellule).

Un isotope radioactif approprié, attaché au stéroïde, permet la destruction spécifique des cellules cancéreuses. En outre, un isotope radioactif approprié permet de visualiser la tumeur par radioimagerie. En fait, ces ligands constituent des agents de pilotage de l'élément actif proprement dit qui est le radionucléide.

Pour des sites de fixation intracellulaire tels que les récepteurs hormonaux, des analogues d'hormones présentant une haute affinité pour le récepteur et une faible affinité pour les protéines de liaison plasmatiques seront les agents de pilotage les plus appropriés.

Un but de la présente invention était en particulier de fournir des complexes du Rhénium et du Technétium présentant une affinité pour le récepteur oestrogène élevée et une bonne stabilité, notamment en vue du marquage de ces complexes par un isotope radioactif de ces métaux pour les applications mentionnées ci-dessus.

EP 441 491 décrit des complexes de technetium et d'estrogène à l'aide de ligands chélatants dérivés de l'acide boronique en position 17α.

EP 345 153, Pure and Applied Chemistry Organometallics, vol. 6, n° 9 pages 1985-7 September 1987 et EP 105 785 décrivent des complexes d'estrogène où soit un ligand L cyclopentadiényle ou phényle est greffé directement sur le carbone en 17α du stéroide, soit un composé LMₓ L'_{y} est complexé sur l'électron pi d'un radical - C ≡ C-.

Les articles de Jour. of Organometallic Chemistry, Vol. 414 n° 1, 1991 pages C22-C27 et organometallics, vol. 11, n° 3, 1992 décrivent des complexes destradiol et de Rhénium avec des composés du type Re₂ (CO)₈(MeCN)₂ dont un des métaux Re est complexé sur l'électron pi de la triple liaison d'un radical ethynyl - C ≡ C-.

La présente invention a en effet pour objet un complexe d'oestrogène organo-métallique répondant à la formule (I). 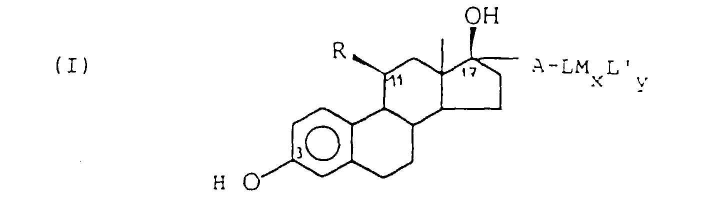 dans laquelle
. A représente un radical alkylène en C₁ à C₇, alcénylène ou alcynylène en C₂ à C₇
. Mₓ représente un ou plusieurs métaux de transition identiques ou différents,
. L'_{y} représente un ou plusieurs ligands identiques ou différents complexant le(s)dit(s) métal(aux) Mₓ, et
. L représente un ligand de Mₓ couplé de façon covalente à A, choisi parmi un groupe phényle ou cyclopentadiényle,
. R représente H ou un alkyl ou alcoxy en C₁ à C₇, éventuellement substitués notamment par un ou plusieurs halogène(s) ; en particulier, R peut représenter H, CH₂Cl, -OCH₃ ou -(CH₂)ₙCH₃ avec n = 1 à 4.

Les fonctions hydroxyles en positions C₃ et C₁₇ confèrent à ces ligands une affinité élevée pour les récepteurs d'oestrogène.

La fonction 11bêta-chlorométhyle, le cas échéant, accentue la stabilité du complexe récepteur-hormone, augmentant ainsi sa concentration à proximité du noyau cellulaire. Cette fonction en position 11bêta assure en fait une liaison quasi-irréversible avec le récepteur oestrogène.

Ces complexes satisfont les conditions requises pour leur utilisation, à savoir la stabilité et la bonne reconnaissance par le récepteur de l'oestradiol.

On observe une amélioration de l'affinité pour le récepteur oestrogène des complexes stéroïdes lorsqu'on éloigne le complexe métallique LMₓL'_{y} du cycle D par le linker A. En particulier, il y a amélioration notoire de l'affinité pour le récepteur oestrogène des complexes stéroïdes cyclopentadiényle-Re(CO)3 en éloignant le cyclopentadiényle du cycle D par un linker, en particulier le groupe CH2 ou le groupe Ethynyle. L'affinité des dérivés stéroïdes cyclopentadiényle Re (CO)3 greffés directement sur le cycle D en 17-alpha est mauvaise.

On peut citer parmi les métaux appropriés du composé organométallique, selon l'invention, les métaux choisis parmi les groupes VI, VII, VIII, et IX de la classification périodique des éléments.

Parmi les ligands L'_{y} de ces composés organométalliques, on peut citer à titre d'exemple CO, CS, CSe, CNR₁, phényle, P(R_{2'}R_{3'} R₄), cyclopentadiényle (Cp), R₁ étant notamment un radical alkyle ou -COR₅ et R₂, R₃, R₄ et R₅ étant notamment des radicaux phényle ou phénoxy substitué ou non, alkyle ou alcoxy en C₁ à C₇ substitué ou non ou bien un atome d'halogène R₅ pouvant être -N(CH₂CH₂Cl)₂.

Les composés MₓL'_{y} peuvent comporter plusieurs métaux, notamment deux métaux, et jusqu'à 12 ligands, notamment 3 à 7 ligands.

Comme on l'a vu, de préférence, le métal Mₓ représente un ou plusieurs atome(s) d'un métal choisi parmi Re ou Tc.

En particulier, dans les complexes selon l'invention, A représente -(CH₂)- ou -(C≡C)-. Dans ce cas, on pourra préparer les complexes par des procédés analogues à ceux décrits dans les exemples qui suivent.

Dans les marqueurs d'affinités de formule I, L est choisi parmi les groupes phényle et cyclopentadiényle.

On peut citer, en particulier, les complexes selon l'invention dans lesquels :
. MₓL'_{y} est choisi parmi Re(CO)₃, Re₂(CO)₇, Tc(CO)₃, ou Tc₂(CO)₇, auquel cas, de préférence, L est couplé de façon covalente à A et L représente Cp ou C₆H₅.

Les composés, selon la présente invention, peuvent être préparés par des procédés connus, notamment par action sur le dérivé de composé oestrogène, d'un dérivé organo-métallique correspondant. Bien entendu, lorsque cela sera nécessaire, certaines des fonctions du composé oestrogène pourront être protégées, en particulier l'hydroxyle en position 3, là encore grâce à des procédés connus.

Ainsi, lorsque A représente -C≡C-, on peut utiliser les réactions suivantes : 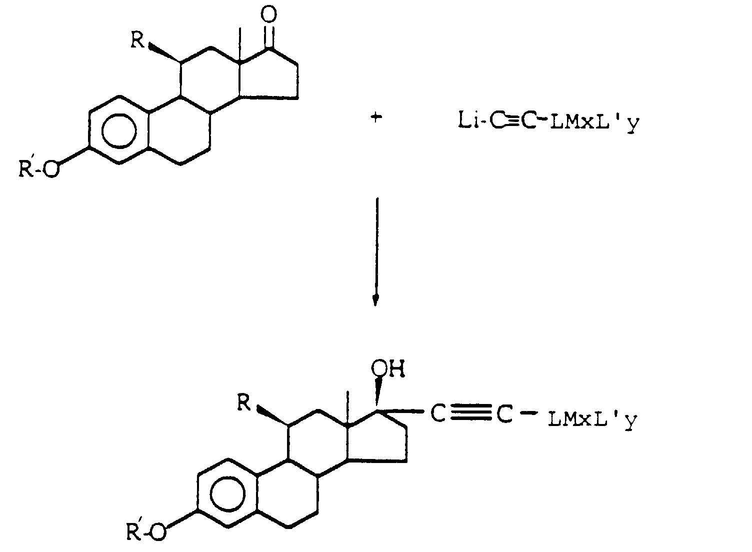 ou la réaction :

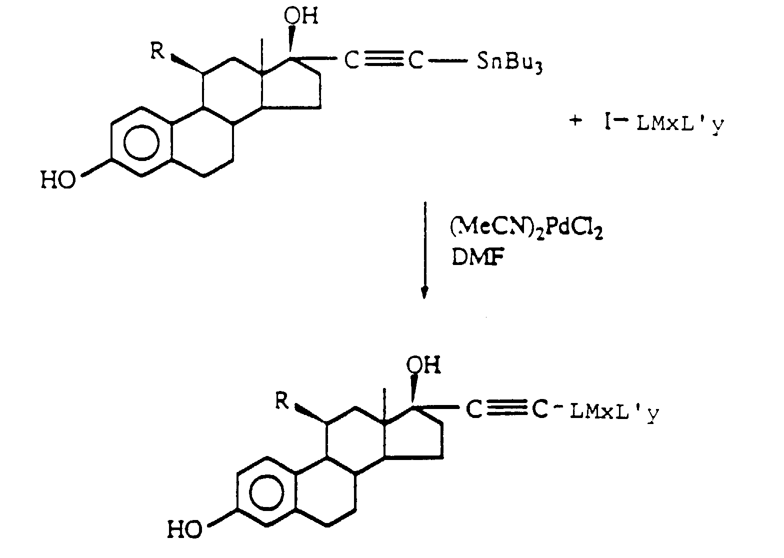

Ce dernier procédé permet de gagner une étape par rapport à celle utilisée précédemment. Ce gain de temps est très important dans le cas où le marqueur radioactif est impliqué.

Lorsque A représente 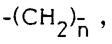 on peut utiliser la réaction suivante :

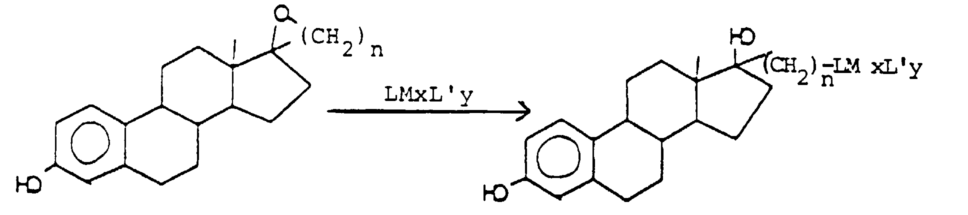

Comme on l'a indiqué, les complexes selon l'invention peuvent être utilisés comme médicaments, notamment des cancers hormono-dépendants, les complexes selon l'invention pouvant être marqués ou non, ou en imagerie médicale lorsqu'ils comportent comme métal un isotope radioactif approprié.

On peut citer en particulier les complexes dans lesquels le métal est ¹⁸⁶Re, ¹⁸⁸Re ou ^{99m}Tc en imagerie médicale et ¹⁸⁶Re ou ¹⁸⁸Re en radiothérapie ciblée in situ.

D'autres avantages et caractéristiques de la présente invention apparaîtront à la lumière des exemples qui vont suivre.

### EXEMPLE 1 : Dérivé 17alpha éthynyl-cyclopentadiényl Rhénium

Il s'agit du complexe d'hormone de rhénium de formule (I)

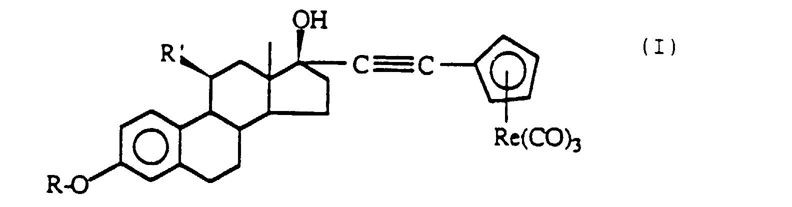

| | | | |
|---|---|---|---|
| Complexe d'hormone | 1 | R = H | R' = H |
| | 2 | R = PhCH₂ | R' = H |
| | 3 | R=H | R'=ClCH₂ |

### I. Voie de synthèse

On a adopté la voie de synthèse suivante selon le schéma 1 suivant :

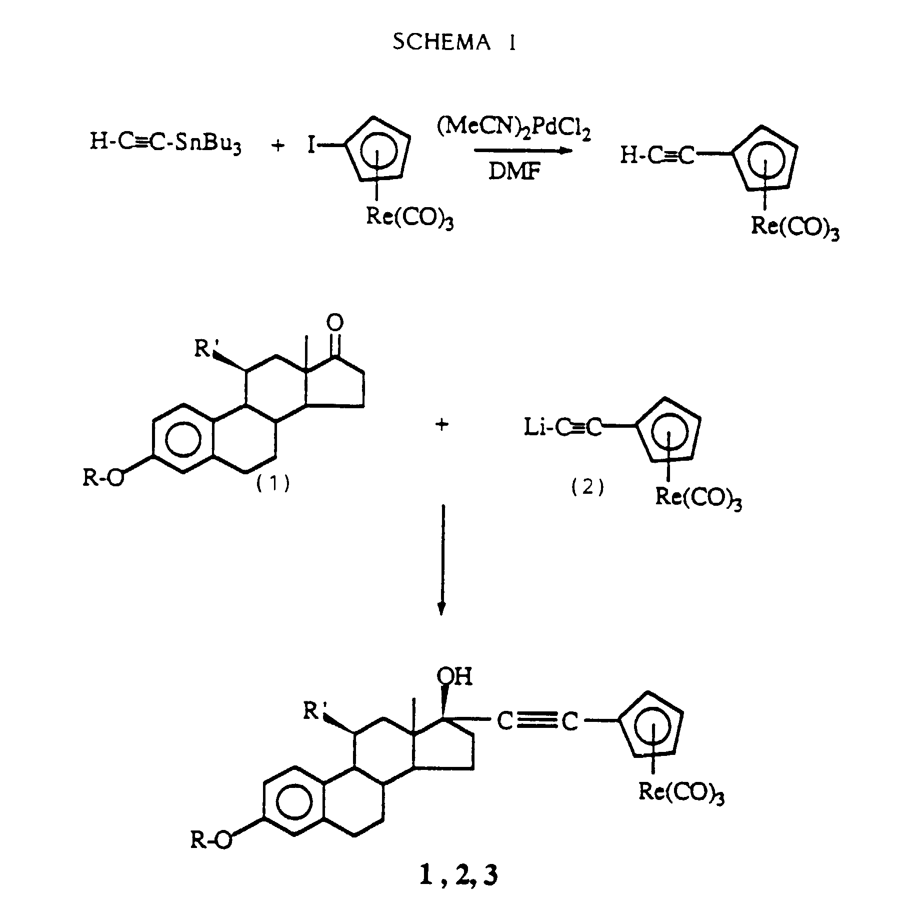

Les complexes d'hormones peuvent être obtenus par la réduction du dérivé de l'oestrone (1) par le lithien Li-C≡C-CpRe(CO)₃(2).

### 1. Synthèse du composé H-C≡C-CpRe(CO)₃

Le composé H-C≡C-CpRe(CO)₃ a été préparé par Stille et al selon la méthode de couplage entre le dérivé d'étain H-C≡C-SnBu₃ et l'iodure ICpRe(CO)₃ (C. Lo Sterzo and J.K. Stille, Organometallics, 1990, 9, 687-694). On a suivi la même méthode pour préparer ce réactif.

H-C≡C-SnBu₃ est obtenu par la réaction entre l'acétylure H-C≡C-Li.EDA et le chlorure ClSnBu₃. Le couplage entre H-C≡C-SnBu₃ et ICpRe(CO)₃ est ensuite réalisé en présence du catalyseur (MeCN)₂PdCl₂.

### 2. Synthèse du complexe d'hormone 2

Dans le but de tester la faisabilité de la méthode, on a d'abord utilisé l'estrone (1) protégé en position 3 par un groupe benzyle. Cette protection permet de supprimer la réaction parasite entre la fonction phénolique et le lithien.

Le lithien Li-C≡C-CpRe(CO)₃ est d'abord généré à -70°C par action de sec-BuLi sur le composé H-C≡C-CpRe(CO)₃. L'oestrone protégé est ensuite ajouté à la solution du lithien maintenu à -70°C. Après le traitement et la purification, on obtient le complexe 2 avec un rendement de 29%.

Il est évidemment possible de déprotéger le complexe d'hormone 2 pour obtenir le complexe d'hormone 1 mais il est plus pratique d'obtenir ce composé directement avec de l'oestrone.

### 3. Synthèse du complexe d'hormone 1

L'utilisation directe de l'oestrone dans ce type de réaction est pénalisée par la réactivité de la fonction phénolique et par sa faible solubilité à basse température. Toutefois, on a réussi à faire une réaction directe sur l'oestrone en transformant au préalable la fonction phénol en phénolate et en diluant le milieu réactionnel.

Le complexe d'hormone 1 est finalement obtenu avec un rendement de 61%.

### 4. Synthèse du complexe d'hormone 3

Le 11bêta-chlorométhyl-bêta-oestradiol présente un très grand avantage du point de vue de l'affinité vis-à-vis du récepteur de l'oestradiol. Sa valeur ARL est très élevée, de l'ordre de 1000. On peut donc espérer que le complexe du 11bêta -chlorométhyl-bêta-oestradiol préserve encore une grande valeur d'ARL.

Le complexe peut se faire selon la même procédure utilisée pour l'oestrone. Etant donné l'accès difficile au 11bêta-chloro-méthyl-bêta-oestradiol et son prix très élevé, il paraît plus astucieux d'ajouter directement l'hormone sur un excès de lithien sans la transformation préalable en phénolate. Dans cette situation, une partie du lithien Li-C≡C-CpRe(CO)₃ réagit avec la fonction phénol mais cette transformation ne présente pas une perte car H-C≡C-CpRe(CO)₃ régénéré est récupérable à la fin de la réaction.

Le complexe d'hormone 3 est obtenu avec un très bon rendement de 78%.

Il est donc possible de préparer les complexes d'hormone de Re présentant une bonne stabilité. Le groupe CpRe(CO)₃ remplit les conditions requises pour un marqueur.

Cette méthode de marquage est applicable pour le marquage au technétium.

D'une manière générale, les composés de rhénium et de technétium se comportent de la même manière vis-à-vis des réactions chimiques. Cette analogie permet un transfert des réactions impliquant des composés de rhénium à la chimie du technétium.

### II. Partie expérimentale

### 1. H-C≡C-SnBu₃

L'acétylure H-C≡C-Li est commerçialement disponible sous la forme stabilisée H-C≡C-Li.EDA. Nous avons donc choisi d'utiliser ce réactif pour la synthèse. 3,5 g ( 0,038 mole ) de H-C≡C-Li.EDA sont mis en suspension dans 40 mL de THF. La solution de ClSnBu₃ dans du THF ( 20 mL ) est ajoutée goutte à goutte à la première solution. On maintient l'agitation pendant une nuit. Après filtration et évaporation du solvant, l'huile obtenue est soumise à une distillation sous vide. On obtient finalement 4,3 g de H-C≡C-SnBu₃ sous forme d'huile incolore qui se distille à 70°C sous 0,1 mm Hg ( littérature 76°C/0,2 mm Hg : Nesmeyanov A. N. et al., Dokl. Akad. Nauk. SSSR, 1976, 174, 96 ). Rendement : 36%. RMN ¹H ( 200 MHz, CDCl₃, δ en ppm ) 2,21 (s, 1H, CH ), 0,92 (t, 9H, Me ).

### 2. H-C≡C-CpRe(CO)₃

Il est préparé selon la méthode décrite par Stille ( C. Lo Sterzo and J. K Stille, Organometallics, 1990, 9, 687-694 ).

(η⁵-IC₅H₄)Re(CO)₃ (0,69 g, 1,5 mmole), H-C≡C-SnBu₃ (0,47 g, 1,5 mmole) et (MeCN)₂PdCl₂ (0,01 g, 0,02 mmole) sont dissous dans 15 mL de DMF. Après une nuit d'agitation, on ajoute 20 mL d'éther et 10 mL d'une solution de KF à 50%. Le mélange est vigoureusement agité pendant 1 h en faisant passer en même temps un courant d'argon. Il est ensuite versé dans une ampoule à décanter. On extrait d'abord le produit par 50 mL d'éther. On lave la solution éthérée par de l'eau ( deux fois ). L'eau de lavage est réunie avec la phase aqueuse précédente, on extrait de nouveau le produit par de l'éther ( deux fois ). Ensuite on réunie ensemble les fractions éthérées et on les lave par de l'eau. Après séchage sur MgSO₄, filtration et évaporation, on obtient un solide que l'on chromatographie sur plaques de gel de silice avec comme éluant : CH₂Cl₂/pentane : 1/10. On obtient finalement le composé H-C≡C-CpRe(CO)₃ sous forme de solide beige, 0,40 g, rdt 73%. RMN ¹H ( 200 MHz, CDCl₃, δ en ppm ) 2,83 (s, 1H, CH ), 5,67 (t, 2H, Cp, J = 2,3 Hz), 5,30 (t, 2H, Cp, J = 2,3 Hz). IR (CH₂Cl₂) ν_{CO} : 2028 F, 1932 F. Ce composé est identifié par comparaison des données IR et RMN avec celles de la littérature (Stille).

### 3. Complexe d'hormone 2

0,216 g (0,6 mmole) de H-C≡C-CpRe(CO)₃ sont dissous dans 4 mL de THF. Après avoir refroidi la solution à -70°C, 0,77 mL d'une solution de sec-BuLi à 1,3 M (1 mmole) sont ajoutés à la solution précédente. Le mélange est agité à -70°C pendant 20 min. On ajoute ensuite goutte à goutte une solution de THF (3 mL) de benzylloxy-3-oestrone (0,216 g , 0,6 mmole). L'addition dure 1/2 h. L'agitation est maintenu pendant une nuit en laissant la température remonter lentement à la température ambiante (15 h). Après hydrolyse, extraction à l'éther et évaporation du solvant, le brut obtenu est chromatographié sur plaques de gel de silice, éluant : éther/pentane : 1/2. On récupère d'abord 0,070 g de H-C≡C-CpRe(CO)₃ non réagi et ensuite 0,120 g de complexe d'hormone 2 sous forme d'huile beige se solidifant dans du pentane. Le rendement est de 29% ou 42% en tenant compte du H-C≡C-CpRe(CO)₃ récupéré. RMN ¹H ( 200 MHz, CD₃COCD₃, δ en ppm ) 7,40 (m, 5H, Ph), 7.19 (d, 1H, H-1, J = 8,4 Hz), 6,77 (dd, 1H, H-2, J = 8,4 et 2,8 Hz ), 6,71 (d, 1H, H-4, J = 2,8 Hz), 5,90 (t, 2H, Cp, J = 2,2 Hz), 5,59 (t, 2H, Cp, J = 2,2 Hz), 5,07 (s, 2H, CH₂-Ph), 4,49 (s, 1H, OH-17), 2,80 (m, 2H, H-6), 0,91 (s, 3H, Me-13). IR (CH₂Cl₂) ν_{CO} : 2025 F, 1930 F. Masse (EI 70 eV) m/z 720 [M]⁺, 692 [M-CO]⁺, 636 [M-3CO]⁺.

### 4. Complexe d'hormone 1

0,216 g (0,6 mmole) de H-C≡C-CpRe(CO)₃ sont dissous dans 4 mL de THF. Après avoir refroidi à -50°C, 0,77 mL d'une solution de sec-BuLi à 1,3 M (1 mmole) est ajoutée à la solution précédente. L'agitation continue pendant 1 h. Dans un autre tube de schlenk, 0,270 g (1 mmole) de l'oestrone sont dissous dans 10 mL de THF. On refroidit la solution à -50°C et on ajoute 0,77 mL de sec-BuLi (1 mmole). La solution reste limpide et incolore. On ajoute ensuite lentement cette solution à la première solution maintenue à - 50°C (1 h). La suite du procédure est identique à celui du complexe 2. Après purification sur plaques, éluant : éther/pentane : 1/1, on obtient le complexe 1 sous forme de solide incolore, 0,225 g, rdt = 61%. F 161°C (éther/pentane). RMN ¹H ( 250 MHz, CD₂Cl₂, δ en ppm ) 7.14 (d, 1H, H-1, J= 8,4 Hz), 6,61 (dd, 1H, H-2, J = 8,4 et 2,1 Hz), 6,55 (d, 1H, H-4, J = 2,1 Hz), 5,71 (s, 1H, OH-3), 5,63 (t, 2H, Cp, J = 2,2 Hz), 5,32 (t, 2H, Cp, J = 2,2 Hz), 2,78 (m, 2H, H-6), 0,89 (s, 3H, Me-13). RMN ¹³C ( 62.89 MHz, CD₂Cl₂, δ en ppm ) 193,29 (CO), 153,23 (C3), 137,92 (C5), 132,07 (C10), 126,06 (C1), 114,80 (C4), 112,27 (C2), 92,71 (C17), 87,52 - 87,45 - 84,04 et 83,95 (4C du Cp), 85,49 et 79,84 (1C du Cp et C≡), 76,54 (C≡), 49,31 (C14), 47,46 (C13), 43,07 (C9), 39,12 (C8), 38,52 (C16) 32,64 (C12), 29,24 (C6), 26,75 (C7), 26,11 (C11), 22,42 (C15), 12,31 (Me-13), IR (CH₂Cl₂) ν_{CO} : 2025 F, 1930 F. Masse (EI 70 eV) m/z 630 [M]⁺, 612 [M-H₂O]⁺, 602 [M-CO]⁺, 546 [M-3CO]⁺.

### 5. Complexe d'hormone 3

Le mode opératoire est identique à celui du complexe 1.

On utilise dans ce cas un excès de Li-C≡C-CpRe(CO)₃ pour neutraliser in-situ la fonction phénolique, 11β-chlorométllyl-oestrone : 0,064g , 0,2 mmole (10 mL THF; H-C≡C-CpRe(CO)₃ : 0,216 g , 0,6 mmole (8 mL THF); sec-BuLi : 0,54 mL, 0,7 mmole (1,3M). La réaction est réalisée à -60°C.

Après une nuit de réaction, on rajoute 20 mL de THF et 0,5 mL d'eau. On filtre ensuite sur gel de silice et on évapore le solvant. Etant donné la faible solubilité du produit formé dans de l'éther ou du dichlorométhane, ce procédé permet de supprimer le problème d'extraction du produit de la phase aqueuse. Le brut obtenu est ensuite chromatographié sur plaques de gel de silice avec comme éluant THF/pentane : 1/3. On isole finalement le complexe 3 sous forme de solide incolore, 0,105 g, rdt 78%. F 219. RMN H (250 MHz, CD₃ COCD₃, δ en ppm) 8,17 (s, 1H, OH-3), 7,06 (d, 1H, H-1 , J = 8,5 Hz), 6,67 (dd, 1H H-2, J = 8,2 et 2,7 Hz), 6,14 (d, 1H, H-4, J = 2,7 Hz), 5,94 (t 2H, Cp, J = 2,2 Hz), 5,59 (t, 2H, Cp, J = 2,2 Hz), 4,63 (s, 1H, OH-17), 3,57 (m, 2H, CH₂Cl), 2,70 (m, 2H, H-6), 1,08 (s, 3H, Me-13), IR(CH₂Cl₂)v_{CO}: 2025. F,1930F. Masse (E1, 70eV)m/z 678(M)⁺, 650 (M-CO)⁺. On récupère également 0,145g de H-C≡C-CpRe (CO)₃ et 0,007 g de 11 bêta-chlorométhyl oestrone.

### EXEMPLE 2

Ces complexes présentent un très grand intérêt du point de vue de marqueur, aussi bien pour le dosage des récepteurs que pour l'imagerie. Toutefois, l'utilisation en imagerie nécessite un court délai de marquage. La réaction ci-dessous est également réalisable par le procédé suivant :

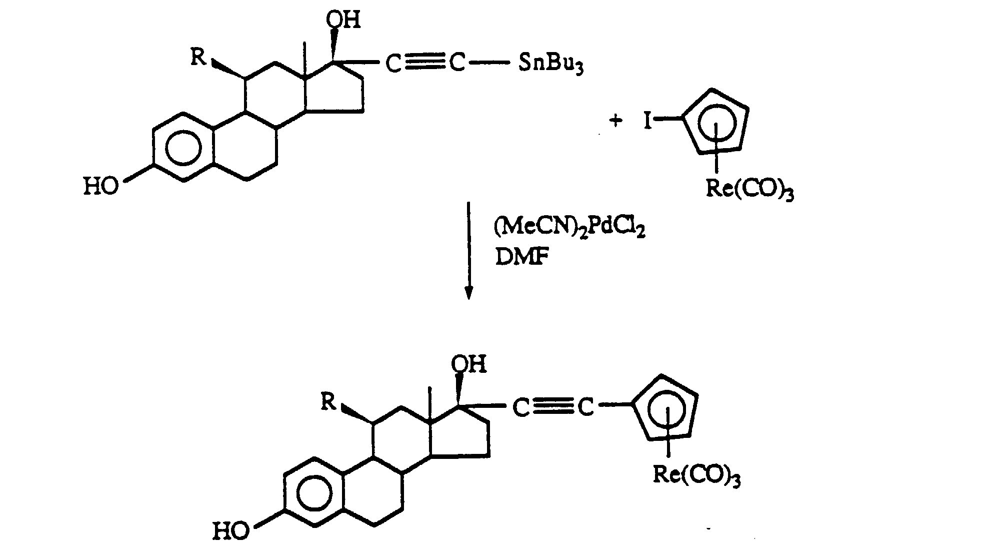

Ce procédure permet de gagner une étape par rapport à celui utilisé dans ce rapport. Ce gain de temps est rès important dans le cas du marqueur radioactif.

### EXEMPLE 3:

### Méthodologie des tests biologiques effectués et résultats

Les valeurs d'affinités relatives de liaison (ARL) ont été déterminées à températures (0° et 25°C) en suivant le protocole suivant : Des fractions de cytosol (surnageant 105.000g) d'utérus de brebis sont incubées (3h à 0°C ; 3h30 à 25°C) en présence de 2 nM d'oestradiol tritié (³H-E₂ et de molarités croissantes du produit que l'on teste (9 molarités comprises entre 10⁻⁹M et 10⁻⁸M. A la fin de la période d'incubation, la séparation des fractions libres et liées d'hormone est réalisée par la technique de précipitation au sulfate de protamine comme décrit dans l'article A. Vessières et al., Biochemistry 1988, 27, 6659. On détermine ensuite la valeur d'ARL qui est le rapport x 100 de la concentration d'oestradiol non radioactif déplaçant 50% de la liaison spécifique de l'oestradiol à son récepteur sur la molarité d'hormone modifiée déplaçant 50% de cette liaison. Plus la valeur de RBA est élevée, plus l'hormone à tester a de l'affinité pour le récepteur de l'oestradiol.

### Résultats

Les trois complexes d'hormones ont été soumis à la mesure de la valeur d'ARL. Les valeurs suivantes ont été trouvées :

| N° du complexe | Complexe | ARL (%) (moyenne de 2 expériences) | |
|---|---|---|---|
| | | 0°C, 3h | 25°C, 3h30 |
| 2 | 17alpha-[-C≡C-CpRe(CO)₃]-3benzyloxy-bêta-oestradiol | 0 | non déterminé |
| 1 | 17alpha-[-C≡C-CpRe(CO)₃]-bêta-oestradiol | 21 | 15 |
| 3 | 17alpha-[-C≡C-CpRe(CO)₃]-11bêta-chlorométhyl-bêta-oestradiol | 29 | 172 |

Le complexe 2 qui ne possède pas de groupement hydoroxyle et position 3 n'est pas reconnu par le récepteur. Pour les complexes du Rhénium, il existe une grande différence de comportement selon la température à laquelle on travaille. A 0°C, les valeurs d'ARL sont très comparables pour les complexes avec ou sans 11bêtachlorométhyl (complexes 3 et 1 respectivement). Par contre, à 25°C, la valeur de ARL du complexe 1 diminue un peu alors que, pour le complexe 3, cette valeur monte à 172%. Cette valeur indique que le complexe est mieux reconnu par le récepteur que l'oestradiol lui-même. C'est la valeur la plus élevée jamais trouvée pour un stéroïde organométallique.

### EXEMPLE 4 :

### Dérivé 17 alpha-méthyl-cyclopentadiényl-Rhénium

Une voie de synthèse se fait selon le schéma 2 ci-après : 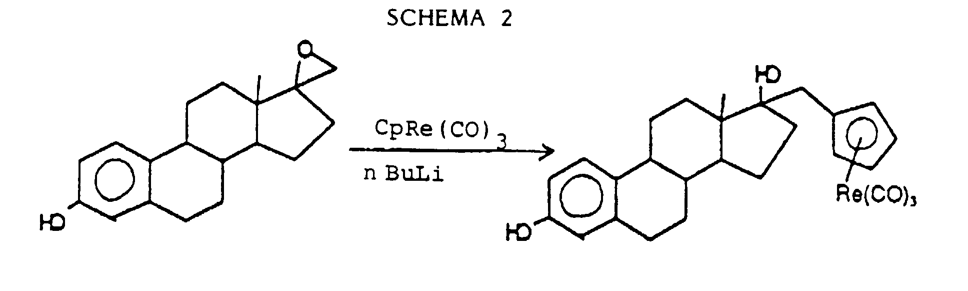 A une solution refroidie à -78°C de CpRe(CO)₃ (177 mg, 5,28 10⁻⁴ mole) dans le THF anhydre (5 ml) est additionné lentement le butyllitium (1,6 M dans l'hexane (0,33 ml). Après une heure d'agitation à -78°C est ajouté l'époxyde 1 (50 mg, 1,76 10⁻⁴ mole) en solution dans le THF anhydre (2 ml). Après 6 heures à température ambiante le milieu réactionnel est hydrolysé avec 10 ml d'eau, extrait avec l'éther (5*10 ml), séché sur MgSO₄, puis concentré sous pression réduite. Leproduit 2 est chromatographié sur plaque éluant éther:pentane 4:6, avec un rendement de 65%.

## Revendications

1. Complexe d'oestrogène organo-métallique répondant à la formule (I). 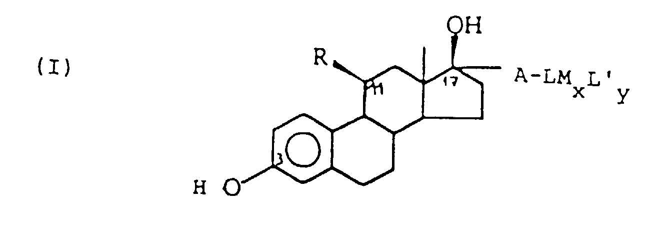 dans laquelle :
. A représente un radical alkylène en C₁ à C₇, alcénylène ou alcynylène en C₂ à C₇
. Mₓ représente un ou plusieurs métaux de transition identiques ou différents,
. L'_{y} représente un ou plusieurs ligands identiques ou différents complexant le(s)dit(s) métal(aux) Mₓ, et
. L représente un ligand de Mₓ couplé de façon covalente à A, choisi parmi un groupe phényle ou cyclopentadiényle,
. R représente H, un alkyl ou alcoxy en C₁ à C₇ éventuellement substitués notamment par un ou plusieurs halogène(s).

2. Complexe selon la revendication 1 caractérisé en ce que Mₓ représente un ou plusieurs atome(s) d'un métal choisi parmi Re ou Tc.

3. Complexe selon la revendication 1 ou 2 caractérisé en ce que A représente -CH₂- ou -C≡C-.

4. Complexe selon l'une des revendications 1 à 3, caractérisé en ce que R représente H, CH₂Cl, -OCH₃ ou -(CH₂)ₙ-CH₃ avec n = 1 à 4.

5. Complexe selon la revendication 4 caractérisé en ce que R est CH₂Cl.

6. Complexe selon l'une des revendications 1 à 5 caractérisé en ce que MₓL'_{y} est choisi parmi Re(CO)₃, Re₂(CO)₇ ou Tc(CO)₃, ou Tc₂(CO)₇ et L représente un cyclopentadiényle.

7. Agent d'imagerie consistant en un complexe selon l'une des revendications 1 à 6, dans lequel le métal est un isotope radioactif approprié pour l'imagerie.

8. Agent d'imagerie selon la revendication 7, caractérisé en ce que le métal est choisi parmi ^{99m}Tc, ¹⁸⁶Re, ou ¹⁸⁸Re.

9. Médicament consistant en un composé selon l'une des revendications 1 à 6.

10. Médicament selon la revendication 9 consistant en un composé selon l'une des revendications 1 à 6 dans lequel le métal est du Rhénium.

11. Médicament selon la revendication 9 ou 10 dans lequel le métal est un isotope radioactif notamment choisi parmi ¹⁸⁶Re ou ¹⁸⁸Re.

## Patentansprüche

1. Organometallischer Oestrogenkomplex der Formel: 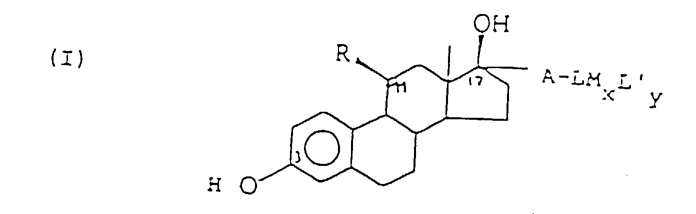 wobei
A ein C₁ bis C₇ Alkylenradikal oder ein C₂ bis C₇ Alkenylen- oder Alkinylenradikal darstellt,
Mₓ eines oder mehrere gleiche oder verschiedene Übergangsmetalle darstellt,
L'_{y} einen oder mehrere gleiche oder verschiedene Liganden darstellt, die das/die Metall(e) Mₓ komplexieren, und
L einen Liganden von Mₓ darstellt, der kovalent an A bindet, ausgewählt aus einer Phenylgruppe oder Cyclopentadienylgruppe,
R H, eine C₁ bis C₇ Alkyl- oder Alkoxygruppe darstellt,
gegebenenfalls bevorzugt substituiert durch ein oder mehrere Halogen(e).

2. Komplex nach Anspruch 1, dadurch charakterisiert, daß Mₓ ein oder mehrere Atom(e) eines aus Re oder Tc ausgewählten Metalls darstellt.

3. Komplex nach Anspruch 1 oder 2, dadurch gekennzeichnet, daß A -CH₂- oder -C-C- darstellt.

4. Komplex nach einem der Ansprüche 1 bis 3, dadurch gekennzeichnet, daß R H, -CH₂Cl, -OCH₃ oder -(CH₂)ₙ-CH₃ mit n = 1 bis 4 darstellt.

5. Komplex nach Anspruch 4, dadurch gekennzeichnet, daß R CH₂Cl ist.

6. Komplex nach einem der Ansprüche 1 bis 5, dadurch gekennzeichnet, daß MₓL'_{y} ausgewählt wird aus Re(CO)₃, Re₂(CO)₇ oder Tc(CO)₃ oder Tc₂(CO)₇ und daß L Cyclopentadienyl darstellt.

7. Markierungsmittel, bestehend aus einem Komplex nach einem der Ansprüche 1 bis 6, wobei das Metall ein für die Markierung geeignetes radioaktives Isotop ist.

8. Markierungsmittel nach Anspruch 7, dadurch gekennzeichnet, daß das Metall ausgewählt wird aus ^{99m}Tc, ¹⁸⁶Re oder ¹⁸⁸Re.

9. Medikament, bestehend aus einer Verbindung nach einem der Ansprüche 1 bis 6.

10. Medikament nach Anspruch 9, bestehend aus einer Verbindung nach einem der Ansprüche 1 bis 6, wobei das Metall Rhenium ist.

11. Medikament nach Anspruch 9 oder 10, wobei das Metall ein radioaktives Isotop ist, bevorzugt ausgewählt aus ¹⁸⁶Re oder ¹⁸⁸Re.

## Claims

1. Organometallic oestrogen complex corresponding to the formula (I): 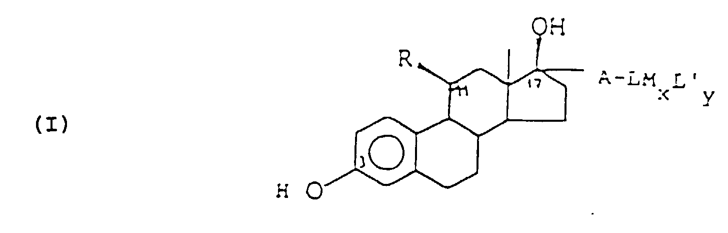 in which:
. A represents a C₁ to C₇ alkylene radical or a C₂ to C₇ alkenylene or alkynylene radical
. Mₓ represents one or more identical or different transition metals
. L'_{y} represents one or more identical or different ligands complexing the said metal(s) Mₓ and
. L represents a ligand for Mₓ coupled covalently to A, chosen from a phenyl or cyclopentadienyl group,
. R represents H or a C₁ to C₇ alkyl or alkoxy, optionally substituted, in particular, with one or more halogen(s).

2. Complex according to Claim 1, characterised in that Mₓ represents one or more atom(s) of a metal chosen from Re and Tc.

3. Complex according to Claim 1 or 2, characterised in that A represents -CH₂- or -C≡C-.

4. Complex according to one of Claims 1 to 3, characterised in that R represents H, CH₂Cl, -OCH₃ or -(CH₂)ₙ -CH₃ with n = 1 to 4.

5. Complex according to Claim 4, characterised in that R is CH₂Cl.

6. Complex according to one of Claims 1 to 5, characterised in that MₓL'_{y} is chosen from Re(CO)₃, Re₂(CO)₇, Tc (CO)₃ and Tc₂(CO)₇ and L represents a cyclopentadienyl.

7. Imaging agent consisting of a complex according to one of Claims 1 to 6, in which the metal is a radioactive isotope suitable for imaging.

8. Imaging agent according to Claim 7, characterised in that the metal is chosen from ^{99m}Tc, ¹⁸⁶Re and ¹⁸⁸Re.

9. Medicinal product consisting of a compound according to one of Claims 1 to 6.

10. Medicinal product according to Claim 9, consisting of a compound according to one of Claims 1 to 6, in which the metal is rhenium.

11. Medicinal product according to Claim 9 or 10, in which the metal is a radioactive isotope chosen, in particular, from ¹⁸⁶Re and ¹⁸⁸Re.
